# EUROPEAN PATENT SPECIFICATION

(11) **EP 2 696 856 B1**
(45) Date of publication and mention of the grant of the patent: **14.09.2016**
(21) Application number: 12706914.4
(22) Date of filing: 16.02.2012
(51) Int. Cl.: A61K 9/16, A61K 9/19, A61K 38/24, A61K 9/00

(54) **CONTROLLED-RELEASE FORMULATION COMPRISING HCG**
RETARD-FORMULIERUNG MIT HCG
FORMULATION À LIBÉRATION CONTRÔLÉE COMPRENANT HCG

(30) Priority: 16.02.2011 IN MM22982010
(43) Date of publication of application: 19.02.2014
(73) Proprietor: Sanzyme Limited, 500034 Hyderabad (IN)
(72) Inventor: PRASAD, Kompella Venkata Subrahmanya, 500034 Hyedrabad (IN); SOMAN, Jay Laxman, 500034 Hyedrabad (IN); VAVIA, Pradeep Ratilal, 400019 Mumbai (IN); VORA, Lalitkumar Khimjibhai, 400019 Mumbai (IN)
(74) Representative: Rees, Kerry
(86) International application number: PCT/IB2012/000272
(87) International publication number: WO 2012/110886

(56) References cited:
- ZHU K J ET AL: "Preparation and characterization of hCG-loaded polylactide or poly(lactide-co-glycolide) microspheres using a modified water-in-oil-in-water (w/o/w) emulsion solvent evaporation technique", JOURNAL OF MICROENCAPSULATION 2001 GB LNKD- DOI:10.1080/02652040010000474, vol. 18, no. 2, 2001, pages 247-260, XP002679345, ISSN: 0265-2048
- ZHANG J X ET AL: "An improvement of double emulsion technique for preparing bovine serum albumin-loaded PLGA microspheres", JOURNAL OF MICROENCAPSULATION 2004 GB LNKD- DOI:10.1080/02652040400008465, vol. 21, no. 7, 2004, pages 775-785, XP002679346, ISSN: 0265-2048

## Description

### FIELD OF INVENTION

The present invention relates to controlled-release drug formulation, in particular a controlled-release formulation comprising human chorionic gonadotropin (hCG) or hCG variant as defined by the claims.

### BACKGROUND OF THE INVENTION

Human chorionic gonadotropin (hCG) is a glycoprotein hormone produced in pregnancy that is produced by developing embryo after conception and later by syncytiotrophoblast (part of the placenta). Its role is to prevent disintegration of corpus luteum of ovary and thereby maintain progesterone production that is critical for pregnancy in humans. hCG may have additional functions; for instance, it is thought that hCG affects the immune tolerance of the pregnancy. Early pregnancy testing, in general, is based on the detection or measurement of hCG. Human chorionic gonadotropin also plays a role in cellular differentiation / proliferation and may activate apoptosis.

Human chorionic gonadotropin is a glycoprotein and composed of 244 amino acids with a molecular mass of 36.7 kDa. It is heterodimeric with an α-subunit identical to that of luteinizing hormone (LH), follicle-stimulating hormone (FSH), thyroid stimulating hormone (TSH), and β (beta) subunit that is unique to hCG. The α-subunit is 92 amino acids long and the β-subunit of hCG gonadotropin contains 145 amino acids and has dimensions. The two subunits create a small hydrophobic core surrounded by a high surface area-to-volume ratio: 2.8 times that of a sphere. The vast majority of outer amino acids are hydrophilic.

Human chorionic gonadotropin interacts with luteinizing hormone/choriogonadotropin (LHCG) receptor and promotes maintenance of corpus luteum during beginning of pregnancy, causing it to secrete hormone progesterone. Progesterone enriches the uterus with a thick lining of blood vessels and capillaries so that it can sustain the growing fetus. Due to its highly-negative charge, hCG may repel the immune cells of mother, protecting the fetus during first trimester. It has also been hypothesized that hCG may be a placental link for development of local maternal immunotolerance.

Various studies suggest that hCG may be a link in development of peritrophoblastic immune tolerance, and may facilitate trophoblast invasion, which is known to expedite fetal development in the endometrium. Because of its similarity to LH, hCG can also be used clinically to induce ovulation in ovaries as well as testosterone production in testes. As a pregnant woman is the most abundant biological source of hCG, some organizations collect urine from pregnant women to extract hCG for use in fertility treatment. As the term progresses, there is a proportional increase in the levels of hCG.

HCG is detectable in the blood serum of approximately 5% of pregnant women by 8 days after conception and in virtually all the rest by 11 days. HCG rises progressively from conception. Levels double on the average, every 30.9 hours until values reach 6500 mIU/ml (6,500 IU/L) at approximately the eighth week after the last menstrual period (LMP). After that the rate of rise becomes individualized, peaking between the 60^{th} and 70^{th} day (9 to 10 weeks) LMP. HCG decreases slightly between the 12^{th} and 16^{th} week post LMP, and then remains constant until birth. The importance of hCG levels for continuing and maintenance of pregnancy is highlighted in Table 1.

There are certain drawbacks of current hCG formulations such as mode of administration of the formulation and number of dosage to be taken by the subject. Despite the usage of the drug for over 4 decades, the principal route of administration remains parenteral that is either intramuscular or subcutaneous. Other routes have been tried but the clinical success rates of such formulations remain an enigma.

Peptides and proteins play a vital role in all biological processes and have received a growing attention in recent years as drug candidates. The rapid advances in peptide and protein pharmacology along with the large-scale production of these compounds by recombinant DNA technology among other techniques have fueled enormous interest in these compounds. Unfortunately, peptide and protein development has far outpaced and advanced more rapidly than the ability to deliver these compounds systemically using convenient and effective delivery systems.

For many applications it is desirable to obtain, sustained and predictable release of a substance. This is especially true in the case of drugs where it is desirable to maintain, a well controlled and stable release rate while avoiding the need for regular administration of the drug, where it is desirable to maintain a well controlled and stable release rate, while avoiding the need for regular administration of the drug.

The main methodologies for obtaining sustained release could be utilizing infusion pumps using hypodermic needles or intubation such as insulin pumps which are mechanical and cumbersome to carry and difficult to maintain, diffusion through membranes such as patches, containing the active drug, compliance is excellent but the range of drugs is limited, iontophoretic methods, quite cumbersome and expensive equipment for delivering the drug and the ideal option, a controlled release formulation, in an injectable form with an administration frequency of 2-3 times a month

Oral route is the most convenient and popular; however most peptide drugs show low oral activity. This is mainly due to degradation by gastrointestinal tract enzymes and poor permeability of the intestinal mucosa. Alternative routes, such as nasal, pulmonary, rectal, buccal, vaginal, and transdermal, have been investigated but with less success.

More than ever before, there has been a significant interest recently in developing novel delivery systems which delivers peptide and protein drugs at a controlled rate throughout extended lengths of time. Instability of proteins can be classified into two types, physical and chemical instability. Physical instability does not involve any covalent modification. It refers to changes in higher order structures such as secondary, tertiary, and quaternary structure. This includes denaturation i.e. unfolding of a protein molecule, adsorption to surfaces, aggregation, and precipitation of proteins or peptides. Chemical instability involves the formation or breaking of covalent bond, leading to new chemical entities. Examples on such pathways are chain cleavage (hydrolysis), deamidation, isomerization, racemization and oxidation.

Nanotechnology could provide optimal vectors able to protect the active ingredient(s) and/or therapeutical biomolecules such as proteins, enzymes or specific polypeptides from degradation (International Journal of Nanomedicine 2010:5 37-49). Synthetic materials for biomedical applications are immediately covered by proteins when put in contact with a biological environment. After protein binding, nanoparticles are quickly cleared by the mononuclear phagocytic system (MPS), also known as the reticuloendothelial system (RES). These macrophages, which are typically Kupffer cells of the liver, cannot directly identify the nanoparticles themselves, but rather recognize specific opsonin proteins bound to the surface of the particles.

Biodegradable non-toxic FDA approved polymers nanosystems are an attractive alternative to liposomes since they have the advantages of longer circulation in the blood stream and generally higher drug carrying capacity. Polymers such as poly (lactic acid) (PLA), poly (lacticco-glycolic acid) (PLGA) have been extensively investigated for their biocompatibility and potential capability of releasing therapeutically proteins in a controlled way even over a prolonged period of time. Regardless of the nanomaterial chosen for protein encapsulation, an important issue that needs to be considered is the understanding of protein-protein interactions (International Journal of Nanomedicine 2010:5 37-49).

PLA and PLGA are FDA-approved as excipients to achieve sustained release of the active ingredient. However, their application in protein delivery systems is often characterized by low entrapment efficiency, burst release, instability of encapsulated hydrophilic protein and partial protein release. To improve the performance of these polymer nanoparticles, polysaccharides such as alginate (ALG) and chitosan (CS) could be applied. CS and its derivatives have been intensively studied as carriers for proteins and drugs. More specifically these nanoparticles can be totally made by CS or used in several copolymer combinations Co-polymers made by the combination of CS/ALG are able to generate a more "friendly" environment which protects peptides and proteins from stressing conditions and allows their stabilization during encapsulation, storage and release.

Although new proteins are available for medical purposes, their administration as therapeutics still remains difficult. Nanosystems seem to be the optimal solution to improve protein bioavailability, biodistribution and safety. Moreover, the combination of nanoparticles with proteins could also be a valid system to achieve the design of efficient nanovectors for drug delivery. Indeed, nanoparticles can be properly tuned for specific applications and could be precisely designed to meet biological needs. It can be anticipated that where large scale fabrication of such nanoparticles is successful, the application of such delivery systems in nanobiotechnology will contribute to de-bottlenecking of current biopharmaceutical manufacture (African Journal of Biotechnology Vol. 7 (25), pp. 4926-4934, 29 December, 2008).

There are several prior art that describes use of biodegradable polymers dissolved in suitable organic solvents for drug delivery. Examples of biodegradable polymers are polylactides, polyglycolides, polycaprolactones, polyanhydrides, polyamides, poly (amino acids), polyvinylpyrrolidone, polyethylene glycol, polyhydroxy cellulose, chitin, and chitosan. The solubility of the polymers in the different solvents will vary depending on their crystallinity, hydrophilicity, hydrogen bonding, and molecular weight (Recent Patents on Drug Delivery & Formulation 2007, 1, 65-71). However, smart bio-degradable polymers and or nanoparticle protein absorption, bioconjugation, and encapsulation are excellent alternatives for ensuring that the SAR of proteins from natural sources such as hCG that are not degraded during the course of administration by parenteral routes.

ZHU K J ET AL 2001 disclosed "Preparation and characterization of hCG-loaded polylactide or poly(lactide-co-glycolide) microspheres using a modified water-in-oil-in-water (w/o/w) emulsion solvent evaporation technique": human chorionic gonadotropin (hCG) entrapped microspheres prepared by a modified w/o/w emulsion solvent evaporation technique.

Human hCG, a gonadotropin is available in both pharmacopoeial and highly purified form as an injectable for either subcutaneous or intramuscular use. However, for ovulation induction a single dose of hCG is injected once the follicle has attained a size of 16 to 20 mm. But for pregnancy maintenance the dosage regimen is complicated and involves injection/s for duration of minimum 8 to 12 weeks, which is cumbersome for pregnant women. The need for repeated injections could be avoided, with controlled release formulation; the required amount of drug for clinical efficacy could be delivered precisely to the site of action i.e. adequate bioavailability.

### SUMMARY OF THE INVENTION

The present invention provides a controlled release formulation comprising human chorionic gonadotropin (hCG) or hCG variant loaded microspheres, wherein the microspheres are preparable by the process comprising (a) emulsifying an aqueous solution of hCG or hCG variant comprising 0.1 to 20% (w/v) gelatin with an organic oily phase comprising 1% to 50% low viscosity grade PLGA to obtain a primary emulsion, (b) emulsifying the primary emulsion with an aqueous phase comprising 0.01% to 30% (w/v) polyvinyl alcohol and 0.1 to 20% sodium chloride to obtain hCG or hCG variant loaded microsphere, (c) hardening the hCG or hCG variant loaded microsphere by stirring at 100-1000 rpm at 25°C to 50°C for 0.5 to 5 hrs, (d) washing the hCG or hCG variant loaded microsphere of step (c) with water, and (e) freeze drying the hCG or hCG variant loaded microsphere of step (d) using 1% to 30% w/w mannitol.

In a preferred embodiment the controlled release formulation of the invention comprises human chorionic gonadotropin (hCG) or hCG variant loaded microspheres having size in the range of 0.02 µm to 500 µm.

In a further aspect of the present invention provides a process for preparation of injectable controlled release human chorionic gonadotrophin (hCG) formulation, said process comprising (a) emulsifying an aqueous solution of hCG or hCG variant comprising 0.1 to 20% (w/v) gelatin with an organic oily phase comprising 1% to 50% low viscosity grade PLGA to obtain a primary emulsion, (b) emulsifying the primary emulsion with an aqueous phase comprising 0.01% to 30% (w/v) polyvinyl alcohol and 0.1 to 20% sodium chloride to obtain hCG or hCG variant loaded microsphere, (c) hardening the hCG or hCG variant loaded microsphere by stirring at 100 - 1000 rpm at 25°C to 50°C for 0.5 to 5 hrs, (d) washing the hCG or hCG variant loaded microsphere of step (c) with water, and (e) freeze drying the hCG or hCG variant loaded microsphere of step (d) using 1% to 30% w/w mannitol.

### BRIEF DESCRIPTION OF THE ACCOMANYING DRAWINGS

Figure 1 shows particle size distribution profile
   A) Example 3, B) Example 4
Figure 2 shows Comparative release profile of hCG from microsphere (Example 1-4)
Figure 3 shows Standard chromatogram of hCG (1mg/ml) in water
Figure 4 shows Standard chromatogram of hCG (1mg/ml) in Phosphate buffer saline (pH 7.4) (Release medium)
Figure 5 shows chromatogram of hCG extracted from microsphere
Figure 6 shows chromatogram of released hCG in 24 hrs from microsphere in PBS buffer (pH 7.4)
Figure 7A) shows standard SDS-PAGE of hCG (reduced form) at various concentrations
Figure 7B) shows hCG stability analysis by SDS-Page, wherein Lane
   1. Molecular Marker
   2. Standard hCG (reduced form)
   3. Standard hCG (non-reduced form)
   4. Dichloromethane treated hCG (Reduced form)
   5. Dichloromethane treated hCG (Non-reduced form)
   6. Dichloromethane treated hCG in presence of gelatin (Reduced form)
   7. Dichloromethane treated hCG in presence of gelatin (Non-reduced form)
   8. HCG loaded microsphere(Reduced form)
   9. HCG loaded microsphere (Non-reduced from)

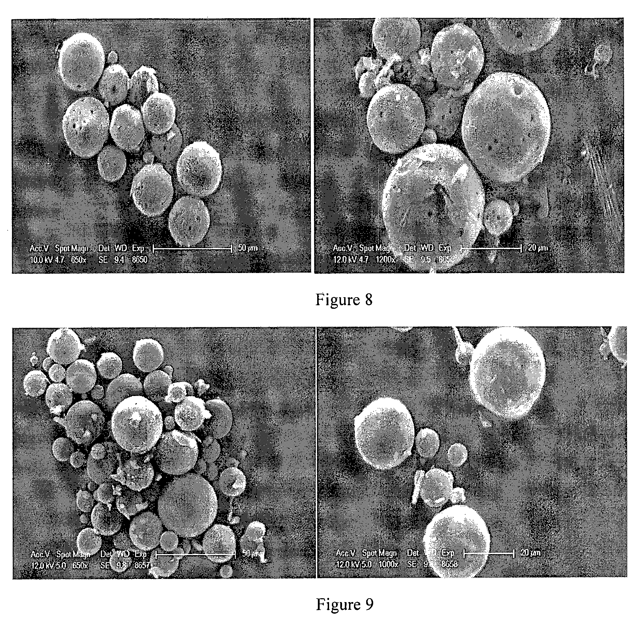

Figure 8 shows scanning electron microscopy of hCG loaded Porous microsphere prepared without Sodium chloride in external water phase Example 2)
Figure 9 shows scanning electron microscopy of hCG loaded Non-porous microsphere prepared with Sodium chloride (0.2M) in external water phase (Example 4)

### DETAILED DESCRIPTION OF THE INVENTION

The present invention provides a controlled-release formulation comprising human chorionic gonadotropin (hCG) or variants thereof and a process for its preparation as per the present claims.

The controlled-release formulation comprising hCG as disclosed in the present invention is stable, effective and advantageous over the hCG formulation known in the art. The controlled-release hCG formulation of the present invention has multiple uses which have not previously existed for therapeutic use. The formulation as disclosed in the present invention is particularly useful in therapeutic regimens for increasing the levels of hCG in maintenance of pregnancy. The novel formulation is filler, taking care of the needs of a stable and preserved solutions, formulations, and products comprising of hCG or hCG variants and using these product in the field of gynaecology.

The controlled-release hCG formulation as disclosed in the present invention comprises hCG encapsulated in polymer matrix, which upon administration releases hCG. The concentration of the hCG in the formulation is in the range of 500 to 100,000 IU.

Encapsulation of the human protein hCG in suitable biodegradable polymers, the controlled release (CR) formulation of this pharmacologically active compound ensures drug delivery to the specific site of action at the therapeutically optimal rate and dosage regimen.

Polymeric nano/microparticles offer specific advantages over other compounds such as liposomes or other carrier by increasing the stability of the drug and its variants, and possess useful controlled release properties. The particle size varies from 10 to 1000 nm. The hCG loaded microsphere as disclosed is prepared by dissolving, entrapping, encapsulating or attached to polymer matrix and depending on the method of preparation microparticles, microspheres, nanoparticles, nanospheres or nanocapsules or a combination of all can be obtained.

The present invention also provides a process of preparation of controlled-release hCG formulation.

Depending on the method of preparation, nanoparticles/microparticles, nanospheres/microspheres or nanocapsules/microcapsules or a combination of all can be obtained.

During polymerization, various stabilizers such as Dextran 10, 40 or 70 or poloxamer -188, -184 or -237 could be used for the process. In addition, surfactants can be used in the controlled-release hCG formulation.

The controlled-release hCG formulation as disclosed in the present invention reduces the number of dosage to be taken, since the subject in needs required to take the formulation once in two weeks or more depending upon necessity, thus avoiding the need for repeated injections.

In another embodiment of the present invention there is provided a controlled-release hCG formulation comprising the encapsulated hCG molecule, wherein the formulation is with or without excipient.

The controlled-release hCG formulation comprises lyophilized and highly purified hCG embedded or encapsulated in nano/micro-particles, nano/micro-capsules or nano/micro-spheres. The carriers are prepared form permitted biodegradable polymers.

In another embodiment of the present invention there is provided a controlled-release hCG formulation, wherein the concentrations of hCG or its variants are ranging from 100 IU to 100,000 IU as Controlled (CR) or Sustained release (SR) formulations (CR/SR).

In another embodiment of the present invention there is provided a controlled-release hCG formulation, wherein the hCG is natural/ synthetic/ or recombinant form.

In another embodiment of the present invention there is provided a controlled-release hCG formulation, wherein the hCG is a variant of hCG.

In one embodiment the ratio of hCG to PLGA used in the process of the present invention is in the range of 1:0.5 to 1:50.

In another embodiment of the present invention the aqueous solution of hCG or hCG variant used in preparing a formulation of the invention may further comprise a stabilizer. The stabilizer may be selected from a group comprising of but not limited to gelatin, dextran, poloxamer, polyols, saccharide, amino acids, organic and/or inorganic salts and combination thereof.

In yet another embodiment the emulsifier used in the process of the present invention selected from a group comprising of but not limited to polyvinyl alcohol, polyvinyl pyrollidone, polysorbate, lecithin, and carboxyl methylcellulose and combination thereof.

In yet another embodiment the aqueous phase used in step (b) of the process of the present invention may comprise an emulsifier and organic/inorganic salt or monosaccharide/oligosaccharide/polysaccharide and derivatives/combinations thereof to obtain hCG or hCG variant loaded microsphere.

Preferably the saccharide is selected from a group consisting of sucrose, dextrose, mannitol and glucose.

In another embodiment step (b) of the process of the present invention may comprises emulsifying the primary emulsion with an aqueous phase comprising an emulsifier and sodium chloride to obtain hCG or hCG variant loaded microsphere.

In a further embodiment the particle size of the hCG or hCG variant loaded microsphere is in the range of 0.02 µm to 500 µm.

In a further aspect of the invention the formulation releases hCG or hCG variant for at least 15 days or more.

Another embodiment of the present invention relates to the controlled release hCG formulation as disclosed in the present invention, wherein the formulation exhibits *in-vitro* rate of release of hCG or hCG variant in the range of 1000IU to 8000IU within 24hrs of administration and 10IU to 1000IU per 24hrs for remaining period.

Another embodiment of the present invention relates to the controlled release hCG formulation as disclosed in the present invention, wherein the formulation exhibits *in-vivo* rate of release of hCG or hCG variant is in the range of 0.1IU/ml to 2.0IU/ml of plasma within 24hrs of administration and 0.0002IU/ml to 0,2IU/ml of plasma for remaining period.

Another embodiment of the present invention provides a controlled release formulation comprising human chorionic gonadotropin (hCG) or hCG variant loaded microspheres as defined in the claims having size in the range of 0.02 µm to 500 µm, wherein the formulation releases hCG or hCG variant for at least 15 days or more.

The novel controlled-release hCG formulation as disclosed in the present invention avoids the need for repeated injections of the highly purified hCG. The NDDS (New Drug Delivery System) allows the protection of the highly purified protein molecules from degradation in circulation. The NDDS technology using the microspheres facilitates controlled and sustained release; it decreases systemic exposure from released molecules; and it decreases the effects of degradation in a biological environment. The nano sized materials can pass directly into tissues and even directly into cells and exert the desired pharmacological activity. The novel formulation disclosed could be administered as a depot injection containing the active drug embedded in the nano particles (nanospheres, nano-capsules, and nano particles).

The currently existing dosage forms are available as ampoules or vials, in a pack consisting of one ampoule or vial of the active lyophilized drug of hCG and the other a solvent for dissolving the active drug along with excipients to be used for intramuscular or subcutaneous injections, whereas the present invention provides the controlled-release hCG formulation which can be administered orally and can be in the form capsule, tablets and various other forms that can be administered orally to a subject in need thereof.

Conventionally nanoparticles are prepared by various methods which include dispersion of the preformed polymers or polymerization of monomers. Several methods to prepare biodegradable nano particles by using either PLA, PLG, PLGA (poly(D,L- surfactant/emulsifying agent like gelatin, poly(vinyl lactide), poly(lactic acid) PLA, poly(D,L-glycolide) alcohol), polysorbate-80, poloxamer-188, etc.)

The process of preparation the novel nanoparticles (microparticle) are relatively easy to implement. Precise amounts of a particular molecule, such as the protein, may be released over a long term. Preparation is preferably carried out under mild, aqueous conditions. The polyelectrolyte layers act as a storage device, and can help inhibit degradation of the "embedded" molecules, for example, by inhibiting proteolysis of peptide drugs. Also, one can avoid high concentrations of the embedded protein molecules in solution, which is advantageous where higher concentrations can lead to deactivation of the payload or where higher concentrations are otherwise undesirable.

Some of the methods commonly used for preparation of the nano/microparticles include, solvent evaporation method, spontaneous emulsification or solvent diffusion method, salting out/emulsification-diffusion method. The latest in the row is the supercritical fluid technology. As per available reports and published peer reviewed articles, during the course of polymerization various stabilizers like Dextran (70/40/10) are used. In additions some surfactants such as polysorbates are used along with the stabilizers. The possibility of using novel hydrophilic polymers is not ruled out in this novel preparation of highly purified hCG and its variants. Dosage:

In woman, about 5000 to 20.000IU of hCG is required to induce ovulation, one injection on 12th day of menstrual cycle, or when the Graffian follicle attains a size > 18mm.

Habitual/Recurrent abortions: once pregnancy is confirmed, 5000 I.U per day is required to be injected on three alternate days. From 9th day of the first injection, 2000 I.U. twice a week, till the 14th week of pregnancy.

In men, about 5000 IU of hCG is required twice weekly for 12 weeks and may have to be continued even upto 1 year to improve semen quality.

### Advantages of the controlled-release Formulation comprising hCG or hCG variant

▪ Using the hCG loaded microsphere daily injection known in the prior art can be reduced to once in 15 days or more.
▪ hCG loaded microsphere as disclosed in the present invention is Stable and effective
▪ Desorption of the surface-bound or adsorbed drug
▪ Diffusion of the drug through the nano/micro-particle matrix
▪ Diffusion through polymer wall (nano/micro-capsules)
▪ Nano/micro-particle matrix erosion and
▪ Combined erosion / diffusion process
▪ Oral hCG formulation cab be formulated using the hCG loaded microsphere as disclosed in the present invention

### Preparation of hCG loaded Microsphere

A w/o/w emulsion solvent evaporation technique was performed for preparation of human Chorionic Gonadotropin (hCG)-loaded poly (lactide-co-glycolide) (PLGA) microspheres. First, human chorionic gonadotrophin (HCG) was dissolved in water or buffer containing 0.5% to 50% (w/v) stabilizer in concentration. Various stabilizers such as gelatin, polyols, sacharides, aminoacidsand/or salt can be used. In the present invention, gelatin (0.1% to 20% w/v) was used as a stabilizer. Low viscosity grade PLGA (poly-dl-lactide-*co*-glycolide.50:50) polymer was dissolved in dichloromethane (DCM). The concentration of PLGA in the organic solvent may vary from 1% to 150% w/v. The preferred concentration is 5% to 50% w/v. To prepare a primary emulsion, aqueous solution of hCG was emulsified with organic oily phase comprising PLGA in high speed homogenizer at 2000 to 24000 rpm, preferably at 20000 rpm for 10 second to 5 minutes, preferably for 30 seconds. Subsequently, the primary emulsion was further emulsified at 1000 rpm to 10000 rpm for 10 seconds to 5 minutes with 10 to 500 fold, preferably 50 to 200 fold of the primary emulsion phase volume with external aqueous phase to obtain a double emulsion comprising the hCG loaded microsphere. Emulsifier can be used in outer aqueous phase, for example polyvinyl alcohol, polyvinyl pyrollidone, polysorbate, lecithin, carboxyl methylcellulose specifically polyvinylalcohol and in the concentration ranging from 0.01 to 30% and preferably 0.02% to 5% w/v. further, 0.1% to 20% salt like sodium chloride, or sugars such as sucrose, dextrose, mannitol, and glucose can be used in outer aqueous phase with emulsifier to get less porous particle. The microspheres were hardened by removing the solvent by means of propeller stirrer, magnetic stirrer, or vacuum evaporator at temperature range from 25°C to 45°C, preferably at 30°C to 40°C for 1 to 5 hours. The hardened microspheres were collected by filtration or centrifugation and washed three to four times with water to remove residual emulsifier and salt. The hCG loaded microspheres thus obtained were mixed with cryoprotactant, such as carbohydrates, polysacharides preferably mannitol (1% to 30% w/w) and freeze dried to get final product. The particle size of the microsphere ranges from 0.02µm to 500µm. Table 2 provides details of the microsphere preparation parameters.

The human chorionic gonadotropin (hCG) as used in the process is highly purified and purity is in range from 5000IU/mg to 20000IU/mg. Ratio of hCG to PLGA can be in the range of 1:50 to 1:0.5.

The present invention is elaborated with the help of following examples. However, the examples should not be construed to limit the scope of the invention. The invention is defined by the claims. Any examples which fall outside the scope of the claims do not form part of the invention but are included for comparative purposes

### Examples

It should be understood that the following examples described herein are for illustrative purposes only and that various modifications or changes in light will be suggested to persons skilled in the art and are to be included within the scope of the appended claims.

### Example 1

A lactic-acid-glycolic acid copolymer (PLGA, Inherent Viscosity 0.16-0.24dl/mg)(100mg) containing lactic acid and glycolic acid in weight ratio 50:50 was dissolved in 1 ml methylene chloride. pHCG (2mg) was dissolved in water (0.1 ml) with 2 mg gelatin as a stabilizer and emulsified with above oily PLGA phase using an ultraturrex (IKA, Germany) homogenizer at 20000rpm for 30sec to obtained water in oil(W/O) emulsion. This W/O emulsion was added to 1% w/w polyvinyl alcohol (PVA) solution. Emulsification was continued using homogenizer (Ultraturrex) at 4000 RPM. This dispersion was gently agitated in 500ml beaker on a magnetic stirring plate containing a stirring bar for 3 to 4 hrs at room temperature. The microspheres were collected by centrifugation at 8000 RPM for 5 min. The obtained microspheres were washed with water for three times and freeze dried with mannitol and stored at 4°C in dessicator. This microsphere was further characterized for encapsulation efficiency and *in*-*vitro* release study (Table 3).

### Example 2

HCG loaded microsphere was prepared according to the same method as described in Example 1 except 3 mg HCG and 10 mg gelatin as stabilizer was used. This microsphere was further characterized for particle size, encapsulation efficiency, and *in-vitro* release study.

### Example 3

HCG loaded microsphere was prepared according to the same method as described in Example 1 except organic solvent contains 0.8ml methylene chloride and 0.2 ml acetone. This microsphere was further characterized for particle size, encapsulation efficiency, and *in-vitro* release study.

### Example 4

HCG loaded microsphere was prepared according to the same method as described in Example 1 except 0.2M (1.16%) sodium chloride was used with 1%w/v polyvinyl alcohol as emulsifier for secondary emulsion preparation. This microsphere was further characterized for particle size, encapsulation efficiency and *in-vitro* release study.

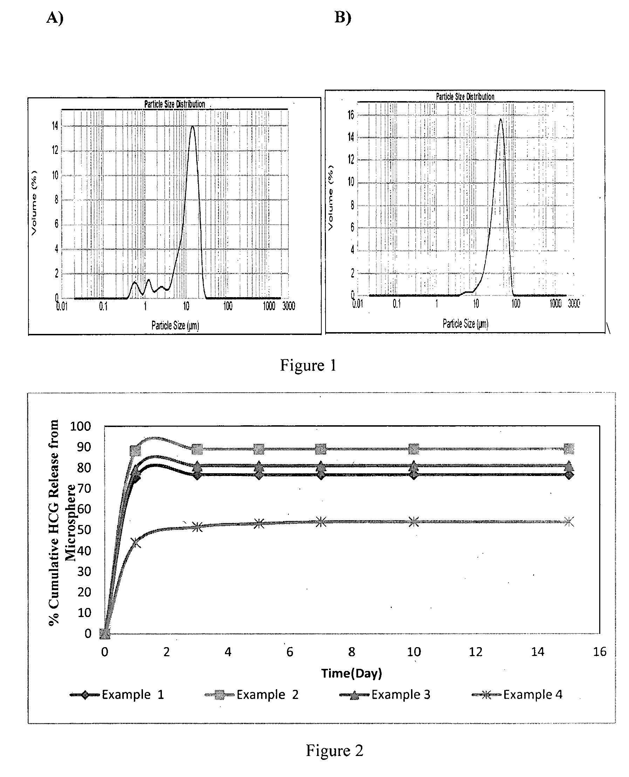

The addition of sodium chloride (NaCl) (1.16% w/v) in external 1% PVA aqueous phase during the secondary emulsion preparation intensity of burst release was reduced from 90%to 43% (Example 4). The decrease in the initial release of hCG in presence of NaCl because of more denser structure of the resulting microsphere. The presence of NaCl in external water phase (high osmotic pressure) during microsphere preparation decrease the solubility of Dichloromethane therefore delayed the polymer precipitation and led to the formation of less porous microsphere. The decrease in porosity reduced the drug accessibility to the release medium and thus related with a lower initial release (Figure 2, Table 3).

### Example 5

### Characterization of hCG-loaded PLGA microsphere

### Particle Size Analysis of hCG-loaded PLGA microsphere

Particle size and Size distribution of hCG-loaded PLGA micro-particles were analyzed using particle size analyzer (Malvern instruments, UK) (Figure 1). Particle size of all examples was found to be in range of 1-60µm with good Polydispersibility (Span Value ∼1). Microsphere preparation in presence of sodium chloride in external water medium gives small particle size (∼20µm). Table 2 provides various sizes of the microsphere obtained.

### Encapsulation Efficiency (EE) of hCG-loaded PLGA microsphere

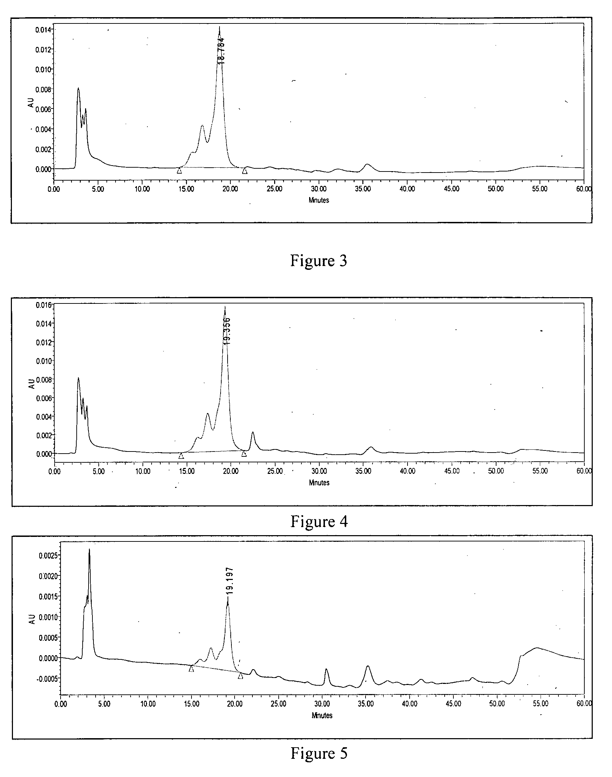

Accurately weighed 5 mg hCG loaded microsphere was dissolved in 200 µl of Acetonitrile. The PLGA was precipitated by adding 800 µl of 10mM Phosphate buffer saline and separated by centrifugation at 16000 RPM for 4 min. The hCG protein supernatant was analyzed by HPLC (waters 717plus autosampler, USA.) Chromatogram of this extracted hCG from microsphere is depicted in Figure 5 compared to standard chromatogram given in Figures 3 and 4. Table 3 provides encapsulation efficiency of the hCG loaded microsphere.

### Scanning Electron microscopy of of hCG-loaded PLGA microsphere

Microsphere preparation without sodium chloride in external water phase shows porous in structure (Figure 8) because of this porous nature of microsphere most of pHCG release in initial period. The addition of sodium chloride (0.2M) in external water phase drastically reduced the porosity of microsphere (Figure 9). The addition of sodium chloride is reduced burst release from 88% to 44% (example 4). The presence of sodium chloride in external water phase during microsphere prepration reduced the water solubility of methylene chloride therefore delayed the polymer (PLGA) precipitation and led to the less porous microspheres and thus lower initial burst release

### Example 6

### Release Profile

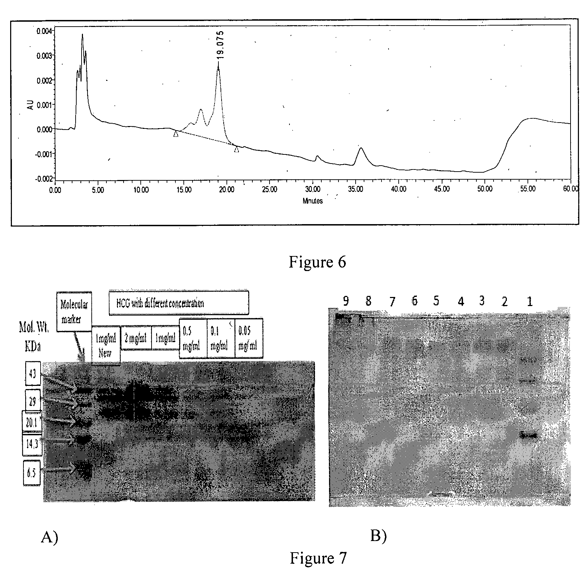

Accurately 10 or 15 mg hCG-loaded microspheres was incubated with 0.8 or 1 ml of 10mM phosphate buffer saline (pH 7.4) containing 0.02% Sodium Azide at 37°C in a temperature-controlled water bath shaker. The aliquots were collected at preselected times by centrifugation for 3 min at 5000 rpm. The HCG content in the supernatant was analyzed using the HPLC (waters 717plus autosampler, USA.) system. Chromatogram of this released HCG is shown in Figure 6. A fresh aliquot of PBS (0.8 ml) was added to the microspheres to replace the removed supernatant.

HCG was found to be release within 24 hours (Burst Release) (Example 1-3) from microsphere. This problem of high burst release may be due to more porous structure of microsphere (Figure 8).

### Example 7

### Analytical Data: SDS-Electrophoresis study for stability analysis of hCG

HCG (R) and HCG Samples were prepared in reduced and non reduced form of 1mg/ml HCG solution respectively. Polyacrylamide gel (15%) was prepared according to standard protocol. 20 µl samples were loaded and run gel at 180Valts in SDS Running buffer. After running, gel was placed in plastic container with fixing solution at room temperature. Pour off fixing solution. Cover with Coomassie blue (0.1%w/v) staining solution was added and shake at room temperature for 2 hours. Pour off staining solution. Gel was washed with 10% acetic acid to destain Samples. In the SDS-Page study, no major degradation of hCG was detected after treating with dichloromethane with and without gelatin as well as in microsphere (Figure 7 A-B).

**Table 1: Serum hCG Levels (LMP - Last Menstrual Period)**

| **Weeks Since LMP** | **Serum hCG Levels (mIU/ml)** |
|---|---|
| 3 weeks | 5-50 |
| 4 weeks | 5-426 |
| 5 weeks | 18-7,340 |
| 6 weeks | 1,080-56,500 |
| 7-8 weeks | 7,650-229,000 |
| 9-12 weeks | 25,700-288,000 |
| 13-16 weeks | 13,300-254,000 |

**Table 2: Particle size of hCG loaded microspheres**

| Example | Particle Size | | | Average Particle size | Span Value |
|---|---|---|---|---|---|
| | D(0.1) (10%) | D(0.5) (50%) | D(0.9) (90%) | Vol. Weighted Mean D[4,3] | |
| 2 | 13.13 µm | 25.02µm | 37.72µm | 25.34µm | 0.98 |
| 3 | 20.µm | 38.48µm | 59.22 µm | 39.17µm | 1.02 |
| 4 | 2.65 µm | 12.41 µm | 19.96µm | 12.1µm | 1.39 |

**Table 3: Encapsulation efficiency and Burst release of different examples**

| Example | Concentration (mg) | | | %Yield | Encapsulation Efficiency (%) | Release within 24 hrs |
|---|---|---|---|---|---|---|
| | hCG | Gelatin | PLGA | | | |
| 1 | 2mg | 2mg | 100mg | 76.1% | 78.86 % | 75.2% |
| 2 | 3mg | 10mg | 100mg | 76.5% | 73.31% | 88.25% |
| 3 | 4.5mg | 8mg | 150mg | 72.5% | 78.92 % | 79.1% |
| 4 | 5mg | 3mg | 100mg | 80.5% | 82.08% | 43.89% |

## Claims

1. A controlled release formulation comprising human chorionic gonadotropin (hCG) or hCG variant loaded microspheres, wherein the microspheres are preparable by the process comprising (a) emulsifying an aqueous solution of hCG or hCG variant comprising 0.1 to 20% (w/v) gelatin with an organic oily phase comprising 1% to 50% low viscosity grade PLGA to obtain a primary emulsion, (b) emulsifying the primary emulsion with an aqueous phase comprising 0.01% to 30% (w/v) polyvinyl alcohol and 0.1 to 20% sodium chloride to obtain hCG or hCG variant loaded microsphere, (c) hardening the hCG or hCG variant loaded microsphere by stirring at 100-1000 rpm at 25°C to 50°C for 0.5 to 5 hrs, (d) washing the hCG or hCG variant loaded microsphere of step (c) with water, and (e) freeze drying the hCG or hCG variant loaded microsphere of step (d) using 1% to 30% w/w mannitol.

2. The controlled release formulation according to Claim 1, wherein ratio of hCG to PLGA is in the range of 1: 0.5 to 1: 50.

3. The controlled release formulation according to Claim 1, wherein the particle size of the hCG or hCG variant loaded microsphere is in the range of 0.02 µm to 500 µm.

4. The controlled relase formulation of any preceding claims wherein the formulation exhibits an *in-vitro* rate of release of hCG or hCG variant in the range of 1000IU to 8000IU within 24 hours of administration and 10IU to 1000IU for at least 15 days thereafter.

5. The controlled release formulation of any of Claims 1 to 3 wherein the formulation exhibits *in-vivo* rate of release of hCG or hCG variant in the range of 0.1 IU/ml to 2.0 IU/ml of plasma within 24 hours of administration and 0.0002IU/ml to 0.2 IU/ml of plasma at least 15 days thereafter.

6. A process for preparation of injectable controlled release human chorionic gonadotrophin (hCG) formulation, said process comprising (a) emulsifying an aqueous solution of hCG or hCG variant comprising 0.1 to 20% (w/v) gelatin with an organic oily phase comprising 1% to 50% low viscosity grade PLGA to obtain a primary emulsion, (b) emulsifying the primary emulsion with an aqueous phase comprising 0.01% to 30% (w/v) polyvinyl alcohol and 0.1 to 20% sodium chloride to obtain hCG or hCG variant loaded microsphere, (c) hardening the hCG or hCG variant loaded microsphere by stirring at 100 - 1000 rpm at 25°C to 50°C for 0.5 to 5 hrs, (d) washing the hCG or hCG variant loaded microsphere of step (c) with water, and (e) freeze drying the hCG or hCG variant loaded microsphere of step (d) using 1% to 30% w/w mannitol.

7. The process according to Claim 6 wherein ratio of hCG to PLGA is in the range of 1: 0.5 to 1: 50.

## Patentansprüche

1. Formulierung mit kontrollierter Freisetzung, die mit humanem chorionischem Gonadotropin (hCG) oder hCG-Variante beladene Mikrosphären umfasst, wobei die Mikrosphären mit dem Verfahren hergestellt werden können, das Folgendes beinhaltet: (a) Emulgieren einer wässrigen Lösung von hCG oder hCG-Variante, die 0,1 bis 20 % (w/v) Gelatine enthält, mit einer organischen öligen Phase, die 1 % bis 50 % PLGA des niedrigviskosen Typs enthält, um eine primäre Emulsion zu erhalten, (b) Emulgieren der primären Emulsion mit einer wässrigen Phase, die 0,01 % bis 30 % (w/v) Polyvinylalkohol und 0,1 bis 20 % Natriumchlorid enthält, um mit hCG oder hCG-Variante beladene Mikrosphäre zu erhalten, (c) Härten der mit hCG oder hCG-Variante beladenen Mikrosphäre durch Rühren bei 100-1000 rpm bei 25°C bis 50°C für 0,5 bis 5 h, (d) Waschen der mit hCG oder hCG-Variante beladenen Mikrosphäre aus Schritt (c) mit Wasser, und (e) Gefriertrocknen der mit hCG oder hCG-Variante beladenen Mikrosphäre aus Schritt (d) mit 1 % bis 30 % w/w Mannitol.

2. Formulierung mit kontrollierter Freisetzung nach Anspruch 1, wobei das Verhältnis von hCG zu PLGA im Bereich von 1:0,5 bis 1:50 liegt.

3. Formulierung mit kontrollierter Freisetzung nach Anspruch 1, wobei die Partikelgröße der mit hCG oder hCG-Variante beladenen Mikrosphäre im Bereich von 0,02 µm bis 500 µm liegt.

4. Formulierung mit kontrollierter Freisetzung nach einem der vorherigen Ansprüche, wobei die Formulierung eine *In-vitro*-Freisetzungsrate von hCG oder hCG-Variante im Bereich von 1000 IU bis 8000 IU innerhalb von 24 Stunden nach der Verabreichung und von 10 IU bis 1000 IU für wenigstens 15 Tage danach aufweist.

5. Formulierung mit kontrollierter Freisetzung nach einem der Ansprüche 1 bis 3, wobei die Formulierung eine *In-vivo*-Freisetzungsrate von hCG oder hCG-Variante im Bereich von 0,1 IU/ml bis 2,0 IU/ml Plasma innerhalb von 24 Stunden nach der Verabreichung und von 0,0002 IU/ml bis 0,2 IU/ml Plasma wenigstens 15 Tage danach aufweist.

6. Verfahren zur Herstellung einer Formulierung mit kontrollierter Freisetzung, die injizierbares humanes chorionisches Gonadotropin (hCG) umfasst, wobei das Verfahren Folgendes beinhaltet: (a) Emulgieren einer wässrigen Lösung von hCG oder hCG-Variante, die 0,1 bis 20 % (w/v) Gelatine enthält, mit einer organischen öligen Phase, die 1 % bis 50 % PLGA des niedrigviskosen Typs enthält, um eine primäre Emulsion zu erhalten, (b) Emulgieren der primären Emulsion mit einer wässrigen Phase, die 0,01 % bis 30 % (w/v) Polyvinylalkohol und 0,1 bis 20 % Natriumchlorid enthält, um mit hCG-oder hCG-Variante beladene Mikrosphäre zu erhalten, (c) Härten der mit hCG oder hCG-Variante beladenen Mikrosphäre durch Rühren bei 100-1000 rpm bei 25°C bis 50°C für 0,5 bis 5 h, (d) Waschen der mit hCG oder hCG-Variante beladenen Mikrosphäre aus Schritt (c) mit Wasser, und (e) Gefriertrocknen der mit hCG oder hCG-Variante beladenen Mikrosphäre aus Schritt (d) mit 1 % bis 30 % w/w Mannitol.

7. Verfahren nach Anspruch 6, wobei das Verhältnis von hCG zu PLGA im Bereich von 1:0,5 bis 1:50 liegt.

## Revendications

1. Formulation à libération contrôlée comprenant des microsphères chargées en hormone chorionique gonadotrophique (hCG) ou en variant de hCG, dans laquelle les microsphères peuvent être préparées par le procédé comprenant (a) l'émulsification d'une solution aqueuse de hCG ou de variant de hCG comprenant de 0,1 à 20% (p/v) de gélatine avec une phase huileuse organique comprenant de 1% à 50% de PLGA à basse viscosité afin d'obtenir une émulsion primaire, (b) l'émulsification de l'émulsion primaire avec une phase aqueuse comprenant de 0,01% à 30% (p/v) d'alcool polyvinylique et de 0,1 à 20% de chlorure de sodium, afin d'obtenir une microsphère chargée en hCG ou en variant de hCG, (c) le durcissement de la microsphère chargée en hCG ou en variant de hCG par une agitation à 100-1000 tours/min à de 25°C à 50°C pendant de 0,5 à 5 h, (d) le lavage de la microsphère chargée en hCG ou en variant de hCG de l'étape (c) par de l'eau, et (e) la lyophilisation de la microsphère chargée en hCG ou en variant de hCG de l'étape (d) en utilisant de 1% à 30% p/p de mannitol.

2. Formulation à libération contrôlée selon la revendication 1, dans laquelle le rapport de la hCG au PLGA se trouve dans la plage allant de 1:0,5 à 1:50.

3. Formulation à libération contrôlée selon la revendication 1, dans laquelle la taille de particules de la microsphère chargée en hCG ou en variant de hCG se trouve dans la plage allant de 0,02 µm à 500 µm.

4. Formulation à libération contrôlée selon l'une quelconque des revendications précédentes, dans laquelle la formulation présente une vitesse de *libération in vitro* de hCG ou de variant de hCG dans la plage allant de 1000 UI à 8000 UI dans les 24 heures après l'administration et de 10 UI à 1000 UI pendant ensuite au moins 15 jours.

5. Formulation à libération contrôlée selon l'une quelconque des revendications 1 à 3, dans laquelle la formulation présente une vitesse de *libération in vivo* de hCG ou de variant de hCG dans la plage allant de 0,1 UI/ml à 2,0 UI/ml de plasma dans les 24 heures après l'administration et de 0,0002 UI/ml à 0,2 UI/ml de plasma pendant ensuite au moins 15 jours.

6. Procédé de préparation d'une formulation injectable à libération contrôlée d'hormone chorionique gonadotrophique (hCG), ledit procédé comprenant (a) l'émulsification d'une solution aqueuse de hCG ou de variant de hCG comprenant de 0,1 à 20% (p/v) de gélatine avec une phase huileuse organique comprenant de 1% à 50% de PLGA à basse viscosité afin d'obtenir une émulsion primaire, (b) l'émulsification de l'émulsion primaire avec une phase aqueuse comprenant de 0,01% à 30% (p/v) d'alcool polyvinylique et de 0,1 à 20% de chlorure de sodium, afin d'obtenir une microsphère chargée en hCG ou en variant de hCG, (c) le durcissement de la microsphère chargée en hCG ou en variant de hCG par une agitation à 100-1000 tours/min à de 25°C à 50°C pendant de 0,5 à 5 h, (d) le lavage de la microsphère chargée en hCG ou en variant de hCG de l'étape (c) par de l'eau, et (e) la lyophilisation de la microsphère chargée en hCG ou en variant de hCG de l'étape (d) en utilisant de 1% à 30% p/p de mannitol.

7. Procédé selon la revendication 6, dans lequel le rapport de la hCG au PLGA se trouve dans la plage allant de 1:0,5 à 1:50.
